# EUROPÄISCHE PATENTSCHRIFT

(11) **EP 0 203 580 B1**
(45) Veröffentlichungstag und Bekanntmachung des Hinweises auf die Patenterteilung: **07.01.1993**
(21) Anmeldenummer: 86107225.4
(22) Anmeldetag: 28.05.1986
(51) Int. Cl.: A61K 37/66

(54) **Gamma-IFN als Wirkstoff zur Hemmung (Verhinderung) von Abbauprozessen im Knochen**
Gamma-IFN as an agent for the inhibition (hindering) of the decay process of bones
Gamma-IFN comme agent d'inhibition (d'empêchement) de processus de décomposition des os

(30) Priorität: 30.05.1985 DE 3519361
(43) Veröffentlichungstag der Anmeldung: 03.12.1986
(73) Patentinhaber: BOEHRINGER INGELHEIM INTERNATIONAL GmbH, 55218 Ingelheim am Rhein (DE)
(72) Erfinder: Peterlik, Meinrad, Prof. Dr., A-1090 Wien (AT); Hoffmann, Oskar, A-1100 Wien (AT)

(56) Entgegenhaltungen:
- EP-A- 0 180 737
- FEBS LETT., Band 185, Nr. 2, Juni 1985, Seiten 287-290, Elsevier Science Publishers B.V.; M. PETERLIK et al.: "Recombinat gamma-interferon inhibits prostaglandin-mediated and parathyroid hormone-induced bone resorption in cultured neonatal mouse calvaria"
- THE JOURNAL OF IMMUNOLOGY, Band 136, Nr. 7, 1. April 1986, Seiten 2478-2481, The American Association of Immunologists, US; M. GOWEN et al.: "Actions of recombinant interleukin 1, interleukin 2, and interferon-gamma on bone resoption in vitro"
- FEBS LETT. Band 180, Nr. 1, Januar 1985, Seiten 43-50, Elsevier Science Publishers B.V.; M.L. STEPHENSON et al.: "Immune interferon inhibits collagen synthesis by rheumatoid synovial cells associated with decreased levels of the procollagen mRNAs"
- BIOCHEMICAL AND BIOPHYSICAL RESEARCH COMMUNICATIONS, Band 120, Nr. 2, 30. April 1984, Seiten 553-558, Academic Press, Inc.; R.L. JILKA et al.: "Inhibition of parathormone-stimulated bone resorption by type 1 interferon"
- JOURNAL OF INTERFERON RESEARCH, Band 4, Nr. 1, 1984, Seiten 135-140, Mary Ann Liebert, Inc., Publishers; O.S. NILSSON et al.: "Effect of interferon on heterotopic new bone formation in mice"
- Pathologie (3), R.Bässler , Böcker et al., S.764-769; Springer-Verlag 1984
- Spezielle Pathologie, Grundmann, S.484-489, 7 Auflage, Urban & Schwarzenberg Verlag (1986)

## Beschreibung

Lebendes Knochengewebe ist einem konstanten Umbauprozeß unterworfen, bei dem sich unter physiologischen Bedingungen Anbau und Abbau im Gleichgewicht befinden. Die Neubildung von mineralisiertem Knochen (Knochenanbau) wird im wesentlichen durch die Aktivität der Osteoblasten bestimmt. Die Aufgabe dieser mesenchymalen Zellen ist die Synthese und Sekretion der einzelnen Komponenten der organischen Knochenmatrix - hauptsächlich Kollagen Typ I - die dann durch Ablagerung von Hydroxylapatit mineralisiert wird. Der Abbau von mineralisiertem Knochen erfolgt durch Osteoklasten, das sind vielkernige Riesenzellen, die wahrscheinlich durch Zellfusion aus Monozyten (Makrophagen) entstehen (1).

Die Aktivität der Osteoblasten und Osteoklasten ist unter physiologischen Bedingungen durch einen komplexen Regulationsmechanismus koordiniert, der das dynamische Gleichgewicht von An- und Abbauvorgängen aufrechterhält. An der Regulation nehmen neben den Hormonen 1,25-Dihydroxyvitamin D₃, Parathormon und (möglicherweise) Calcitonin (2) auch verschiedene lokale Mediatoren und Gewebshormone (3), insbesondere Prostaglandine (2), und noch nicht identifizierte "coupling factors" (4) teil. Letztere werden dafür verantwortlich gemacht, daß es nach Stimulierung der Osteoklastenaktivität reaktiv auch zu einer erhöhten Aktivität der Osteoblasten kommt, was z.B. zur Folge haben kann, daß ein erhöhter Abbau durch einen vermehrten Knochenanbau wieder kompensiert werden kann. Störungen dieses Gleichgewichtes können zu mannigfaltigen Osteopathien führen, von denen besonders eine, die Osteoporose, bei der es - hauptsächlich im fortgeschrittenen Lebensalter - zu einem Schwund der Knochenmasse, d.h. des mineralisierten Anteils und der organischen Knochenmatrix kommt, der weit über das dem Alter entsprechende Ausmaß hinausgeht. Die Osteoporose stellt wahrscheinlich die häufigste Knochenerkrankung dar.

Insbesondere werden Frauen nach der Menopause von dieser Krankheit betroffen. So haben z.B. Untersuchungen in den USA ergeben, daß 25 % aller Frauen im Alter über 65 Jahre an dieser Erkrankung leiden (5). Nach vorsichtigen Schätzungen rechnet man in der Bundesrepublik Deutschland mit etwa drei bis vier Millionen Betroffenen. Die Folge des Knochenschwundes sind vermehrte Knochenbrüche, z.B. der Wirbel, des Oberschenkelhalses oder der Handknochen, Teile also, die besonders stark beansprucht werden. Diese Frakturen verursachen oftmals schwere und bleibende Bewegungseinschränkungen und Behinderungen. Eine typische Veränderung ist die Krümmung der Wirbelsäule. Hierdurch wird die Beweglichkeit des Patienten beeinträchtigt. Neben ständigen Schmerzen hat die gekrümmte Haltung aber auch Auswirkungen auf Herz und Lunge, sowie auf den Magen-Darm-Trakt.

Beteiligt an dieser Krankheit sind vor allem der Calziumhaushalt und verschiedene Hormone, die diesen und damit auch die Mineralisierung des Knochens sowie die Neubildung der organischen Knochenmatrix steuern. In dieser Hinsicht kommt besonders den Östrogenen, die bei Frauen in der Menopause abnehmen, eine große Bedeutung zu.

Was die spezifischen Wirkungen der einzelnen Hormone und Mediatoren betrifft, so steht eindeutig fest, daß Parathormon und Vitamin D, besonders sein in der Niere gebildeter Metabolit 1,25-Dihydroxyvitamin D₃, in erster Linie eine Hemmung der Osteoblastenaktivität bewirken (cf. 6). Zusätzlich scheinen sie die Fusion von monozytenartigen Precursor-Zellen zu erleichtern und damit zu einer Erhöhung der Zahl der Osteoklasten im Knochengewebe beizutragen (cf. 7). Eine direkte Beeinflussung deren Aktivität kann nicht ausgeschlossen werden.

Auch Prostaglandine bewirken - durch einen anderen Mechanismus - eine Erhöhung der Zahl und Aktivität von Osteoklasten (8).

In letzter Zeit hat sich herausgestellt, daß auch Interleukine und Lymphokine eine bestimmte Rolle im Prozeß des Knochenumbaus spielen können (1). Interleukin 1, das im Rahmen der Immunantwort von Makrophagen gebildet wird, beschleunigt den Abbau von Knochen in Organkultur (9), wobei ein Teil seiner Wirkung darauf beruht, daß die endogene Synthese von osteolytisch wirkenden Prostaglandinen stimuliert wird (9). Der sogenannte "Osteoclast Activating Factor" (OAF) ist ein Lymphokin, das von T-Lymphozyten als Antwort auf verschiedene Stimuli - unter anderem auch Interleukin 1 (vgl. 1) - produziert wird. Der OAF hat ebenfalls eine osteolytische Wirkung, die durch Prostaglandine vermittelt wird (10).

Die Behandlung der Osteoporose ist schwierig, denn ein Knochensubstanzverlust ist zur Zeit praktisch nicht mehr rückgängig zu machen; der Wiederaufbau durch Fluoride ist umstritten. Ziel der Therapie muß es daher sein, das Fortschreiten der Erkrankung zu verhindern, indem die Knochenabbauprozesse auf das Ausmaß der Knochenneubildung reduziert werden.

Wichtig ist aber auch, der Krankheit vorzubeugen. In den USA wird den Frauen empfohlen, mit der Nahrung ausreichend Calzium aufzunehmen und zwar bereits vor der Menopause.

Seit einiger Zeit ist bekannt, daß auch Calcitonin, ein Peptidhormon, den Knochenabbau hemmen kann. Es hemmt vor allen Dingen die Wirkung des Parathormons auf die Osteoklasten, doch scheint es darüberhinaus auch eine eigenständige suppressorische Wirkung auf die Osteoklastenaktivität zu haben (2). Die Calcitonin-Behandlung ist jedoch aufwendig und ist empfehlenswert wohl nur bei Patienten, bei denen der Knochenschwund schon weit fortgeschritten ist. Außerdem scheint sie nur zeitlich begrenzt sinnvoll zu sein, da der Organismus nur vorübergehend auf Calcitoningaben reagiert ("Escape"-Phänomen).

Aufgabe der vorliegenden Erfindung war es daher, ein Mittel zur Behandlung von durch Knochenschwund hervorgerufenen Erkrankungen des Skeletts bereitzustellen.

Überraschenderweise wurde nun gefunden, daß γ -Interferon eine äußerst starke regulierende Wirkung auf die beim Knochenauf- und -abbau ablaufenden Prozesse ausübt.

γ-Interferon (IFN₋γ ), auch als Immuninterferon bezeichnet, wird wie Interleukin 1 ebenfalls zu den Lymphokinen gerechnet, weil es auf Lymphozyten entweder nach spezifischer oder unspezifischer Stimulierung durch Antigene,gegen die die Lymphozyten vorher sensibilisiert worden waren, gebildet wird. Es wurde vermutet, daß IFN-γ die Verschmelzung von Monozyten in Osteoklasten fördern könnte, weil sich herausgestellt hatte, daß IFN-γ in der Kultur von humanen Blutmonozyten die Entstehung von mehrkerningen Riesenzellen (Polykaryonen) fördert (11). Außerdem kann IFN-γ Makrophagen aktivieren, die dann selber wieder osteolytische Aktivitäten entfalten können (s.o.)

Es wäre zu erwarten gewesen, daß γ-Interferon den durch Osteoklasten bewirkten Abbau von mineralisiertem Knochen fördern würde. Umso überraschender ist daher die hemmende Wirkung auf die osteolytischen Mechanismen zu beurteilen.

Gegenstand der Erfindung ist die Verwendung von γ-IFN zur Herstellung eines Arzneimittels mit regulierender Wirkung auf die beim Knochenauf- und -abbau ablaufenden Prozesse.

Die vorliegende Erfindung beschreibt erstmals, daß IFN-γ überraschenderweise in der Lage ist, Vorgänge, die zu krankhaftem Knochenschwund führen, zu hemmen. Dies erfolgt in erster Linie durch eine Unterdrückung der endogenen Synthese von osteolytisch wirkenden Prostaglandinen im Knochen durch Interaktion des γ-Interferons mit dem Prostaglandinsynthetasekomplex (Cyclooxygenasesystem). Diese Wirkung besitzt das Calcitonin nicht.

Diese Inhibition der Prostaglandinsynthese im Knochen ist überraschenderweise eine für IFN-γ spezifische Wirkung! Ein derartiger Effekt konnte für IFN- α,β nicht nachgewiesen werden. Eine zweite Wirkung ähnlich der des Calcitonins (7) besteht in einer Hemmung der durch Parathormon stimulierten Osteoklastenaktivität. Dieser Effekt des IFN-γ wird allerdings erst in einem höheren Konzentrationsbereich nachweisbar. Die Calcitonin-artige Wirkung dürfte allerdings auch anderen Interferontypen zukommen, da z.B. Jilka und Hamilton gezeigt haben, daß menschliches Leukozyteninterferon die durch PTH induzierte Knochenresorption in der Organkultur zu hemmen vermag (19).

Die Hemmung der Prostaglandinsynthese durch γ-Interferon ist völlig überraschend, da man bisher annehmen mußte, daß Interferone die Prostaglandinsynthese eher stimulieren (20). So konnte z.B. das bei der Therapie mit α- und β-Interferonen auftretende Fieber auf eine Erhöhung der PGE₂-Konzentration im Hypothalamus zurückgeführt werden (21).

Die Erklärung für diese überraschende Wirkung des γ-Interferons auf die Osteolyse könnte in einer besonderen Spezifität des Cyclooxygenasesystems des Knochens liegen. Unterschiede in der Gewebsspezifität des Prostaglandinsynthetasekomplexes sind seit langer Zeit bekannt (22). Im Knochen scheint dieser Enzymkomplex für IFN-α und IFN-β nicht sensitiv zu sein. Außer den vorliegenden Ergebnissen (s.Beispiel 4) spricht auch die Tatsache dafür, daß in der erwähnten Studie von Jilka und Hamilton (19) keinerlei Einfluß des Typ 1 Interferons auf die basale Knochenresorption gefunden wurde. Ein weiterer derartiger Hinweis auf die unterschiedliche Sensitivität des Enzymkomplexes kann den Untersuchungen von Nilsson et al. entnommen werden, bei denen die Auswirkung von Mausinterferon aus C-243 Zellen auf heterotope Knochenimplantate in Mäusen untersucht wurde (23). Es ergaben sich dabei keinerlei Hinweise auf eine eventuelle osteolytische Wirkung dieses Interferons, was zu erwarten gewesen wäre, wenn das verwendete Interferon die Prostaglandinsynthese auch im Knochen (so wie im Hypothalamus) stimulieren würde.

Ein weiterer Gegenstand der Erfindung ist die Verwendung der pharmazeutischen Zusammensetzung mit γ-Interferon als Wirkstoff zur Behandlung von Erkrankungen, die durch pathologisch veränderten, lokalen oder generalisierten Knochenabbau hervorgerufen werden, wie beispielsweise die Osteoporose in all ihren Formen und Stadien.

Die Äthiologie und Pathogenese der senilen Osteoporose sind zwar weitgehend unklar, doch wird übereinstimmend angenommen, daß es durch ein relatives Überwiegen von Abbauvorgängen zu einer pathologischen Rarefizierung der Knochenmasse kommt (5). Auffallend dabei ist die Tatsache, daß Patienten mit Osteoporose keine bemerkenswerten Veränderungen, was die Serumspiegel der knochenresorbierenden Hormone PTH und 1,25-Dihydroxyvitamin D₃ betrifft, aufweisen.

Es ist daher zu vermuten, daß die erhöhte Knochenabbaurate vor allem auf die Wirkung lokaler Faktoren zurückgeführt werden könnte. Hinweise dafür finden sich in einer Untersuchung von Atik et al.(26), die in Knochenbiopsien von Patienten mit seniler Osteoporose eine höhere PGE₂-Aktivität als bei Kontrollpersonen gefunden haben. Fujik et al. (27) haben bei Patienten mit Osteoporose eine Veränderung des Verhältnisses von T-Helfer- zu T-Suppressor-Lymphozyten festgestellt und daraus geschlossen, daß eine gestörte Immunregulation bei Osteoporose möglich sei; das ist insofern von Bedeutung, als gerade die immunregulierenden Faktoren Interleukin 1 und Osteoclast Activating Factor - wie bereits ausgeführt - zu Stimulatoren der PG-Synthese gehören. Die überraschende Hemmwirkung von IFN-γ auf die PG-Synthese im Knochen bietet insbesondere Vorteile bei der Therapie der Osteoporose, wenn man in Betracht zieht, daß IFN-γ in ähnlicher Weise wie Calcitonin die Knochenresorption beeinflußt und daß gerade dieses Hormon in der Osteoporosetherapie in vielen Fällen eine gute Wirkung gezeigt hat (28). Wie bereits gesagt, besitzt das Calcitonin jedoch keine Wirkung auf die PG-Synthese; sein Wirkungsmechanismus ist ein anderer.

Diese Unterschiede ergeben die Möglichkeit, je nach Fall zu entscheiden, welches der beiden Mittel vorteilhafterweise einzusetzen ist oder ob beide gemeinsam einzusetzen sind.

Die überraschende Hemmung der Prostaglandinsynthese durch IFN-γ macht es zum Gegenspieler des Interleukin 1 und des Osteoclast Activating Factors (OAF) im Prozeß des Knochenumbaues. Diese Eigenschaft ist therapeutisch von großer Bedeutung, da die osteolytische Wirkung von Prostaglandinen, deren Synthese durch verschiedene Entzündungsmediatoren und nicht zuletzt durch den OAF bei Erkrankungen des rheumatischen Formenkreises, insbesondere bei der rheumatoiden Arthritis und ähnlichen Erkrankungen stimuliert wird, zu den bei dieser Erkrankung beobachteten Desstruktionen des Gelenkknochens trägt. Gegenstand der Erfindung ist daher auch die Verwendung des erfindungsgemäßen Mittels bei der Behandlung von Erkrankungen des rheumatischen Formenkreises, insbesondere der rheumatoiden Arthritis.

Eine entsprechende Therapie mit IFN-γ ist insofern besonders vorteilhaft gegenüber einer Behandlung mit nichtsteroidalen Entzündungshemmern (z.B. Indomethacin), da gerade diese Substanzen wegen ihrer Hemmwirkung auf die PG-Synthese auch in anderen Geweben (z.B. Magenschleimhaut) schwerwiegende Nebenwirkungen (akute Ulcera) aufweisen. Von manchen nicht-steroidalen Antiphlogistika wird aber auch nur eine geringe Hemmwirkung auf die PG-Synthese angegeben; hier könnte sich eine Kombination mit γ-IFN wegen dessen selektiver Wirkung auf die PG-Syntese im Knochen als vorteilhaft erweisen. Es läßt sich allerdings schwer abschätzen, welche Auswirkungen eine Interferontherapie auf den Verlauf einer primär chronischen Polyarthritis (rheumatoide Arthritis) haben könnte, da bei dieser Erkrankung eine Vielzahl von Autoimmunphänomenen auftritt, deren mögliche Beeinflussung durch die immunregulatorische Wirkung von IFN-γ nur ungenügend erforscht ist (24). In Abhängigkeit von der verwendeten Dosis kann IFN-γ sowohl eine hemmende als auch stimulierende Wirkung auf die Produktion von Immunglobulinen durch B-Lymphozyten haben. Bei verschiedenen Immunerkrankungen wurden tatsächlich Interferone in Patientenseren festgestellt. Es hat jedoch den Anschein, daß diese IFN-Aktivitäten eher einem veränderten IFN-α als einem IFN-γ zuzuschreiben sind. Was die zellvermittelte Immunität betrifft, so wirkt IFN-γ im allgemeinen hemmend auf die Proliferation von T-Lymphozyten und kann so Phänomene der zellvermittelnden Immunität wie Transplantatabstoßung oder Überempfindlichkeitsreaktionen vom verzögerten Typ unterdrücken, was bei Behandlung der PCP von Vorteil wäre; andererseits kann IFN-γ aber unter bestimmten Bedingungen eine Stimulierung der zellulären Immunantwort bewirken (24).

Zusätzlich zur bereits bekannten Möglichkeit, das Tumorwachstum zu unterdrücken, bietet sich die Fähigkeit von IFN-γ , die Prostglandinsynthese im Knochen zu supprimieren, auf jeden Fall vorteilhaft bei einer Behandlung von den Tumoren an, die zur Metastasierung in den Knochen und zum Knochenabbau beitragen, weil sie selbst Prostaglandine produzieren oder die Prostaglandinsynthese im Knochen stimulieren (vgl. dazu 25).

Die gezeigte Wirkung des γ-Interferons auf Prozesse, die beim Knochenauf- und -abbau ablaufen, machen γ-Interferon also überraschenderweise zu einem hervorragenden Wirkstoff für die Herstellung von Arzneimitteln; die bei der Behandlung von systemischen oder lokalen Knochenerkrankungen mit entzündlicher oder auch nicht-entzündlicher Genese (degenerative Erkrankungen), die durch Hemmung des Knochenaufbaues oder durch erhöhte Resorption des Knochens hervorgerufen werden, eingesetzt werden können.

Hierzu gehören neben den bereits genannten ganz allgemein verschiedene durch Parathormon hervorgerufene Osteodystrophien, insbesondere renal bedingte Osteopathien. Bekannt ist die bei progredienter Niereninsuffizienz durch Parathormon hervorgerufene Knochenresorption. Bei einem Teil dieser Erkrankungen beobachtet man autoimmun bedingte Glomerulonephritiden. Gerade in solchen Fällen ist γ-IFN als Wirkstoff mit immunsuppressiven Eigenschaften hervorragend als Therapeutikum geeignet. Das erfindungsgemäße Mittel hemmt die Knochenresorption und beeinflußt wirksam die Autoimmunreaktion.

Ebenso ergibt sich die Behandlung einer anderen Osteodystropie, die Morbus Paget mit dem erfindungsgemäßen Mittel.

Weiterhin bietet γ-IFN den Vorteil, in all den Fällen, bei denen im Verlaufe einer Calcitonintherapie mangelnde Ansprechbarkeit und/oder Immunreaktionen auftreten, eingesetzt werden zu können.

Auch möglich ist die Kombination des Mittels mit Calcitonin oder mit anderen zur Behandlung von Knochenerkrankungen bekannten Mitteln, da γ-IFN wie gezeigt, durch seinen anderen Wirkungsmechanismus eine zusätzliche Wirkung aufweist.

Zur Behandlung der bereits oft in jungen Jahren auftretenden Kiefer- und Zahnerkrankungen mit gesteigertem Knochenschwund wie z.B. alveoläre Knochenresorptionen, die allgemein als periodontale Erkrankungen bezeichnet werden, ist das Mittel ebenfalls hervorragend geeignet.

Das Mittel kann den zu behandelnden Patienten oder auch Säugetieren systemisch oder auch lokal, beispielsweise intraartikulär verabreicht werden, wobei generell die orale, topische, parenterale und auch buccale Verabreichung möglich ist. Prinzipiell sind alle die Darreichungsformen geeignet, die eine Erhöhung des γ-Interferon Plasma-/Gewebespiegels bewirken, beispielsweise die für γ-IFN üblichen Lösungen. Möglich sind jedoch auch andere Dosierungsformen.

In überraschender Weise sind bereits geringe Mengen an γ-IFN ausreichend für die erfindungsgemäße Verwendung. Dosierung und Dosierungsrate können ähnlich denen sein, die zur Zeit bei klinischen Prüfungen für γ-IFN gelten. Je nach Applikationsort kann die Dosierung variiert werden.

Entscheidend ist, daß die Dosierung so gewählt wird, daß ein wirksamer Anstieg des γ-Interferon Plasma-/Gewebespiegels erreicht wird.

Die Mittel können übliche pharmazeutische Hilfs- und/oder Trägerstoffe und/oder Stabilisierungsmittel enthalten.

Als mögliche Stabilisatoren seien beispielhaft Aminosäuren, Di-, Tri- und Tetrapeptide, Zucker oder Albumine genannt.

Dem Fachmann sind eine Vielzahl an Hilfs-, Trägerstoffen und Stabilisierungsmittel bekannt und auch wie diese mit dem Mittel formuliert werden können. In diesem Zusammenhang sei ausdrücklich auf die bei E. W. Martin in Remington's Pharmaceutical Sciences beschriebenen Stoffe und ihre Formulierung verwiesen.

### Legende zu den Abbildungen

- Abbildung 1:: Einfluß von IFN-γ auf die Calciumabgabe aus kultivierten neonatalen Mauskalvarien (basale Knochenresorption).
- Abbildung 2:: Einfluß von IFN-γ auf die Knochenresorption, stimuliert durch PGE₂, Thrombin oder Arachidonsäure (AA).
- Abbildung 3:: Einfluß von Salm- Calcitonin (SCT) auf die Thrombin-induzierte Resorption von kultivierten neonatalen Mauskalvarien.
- Abbildung 4:: Einfluß von Salm-Calitonin (SCT) auf die Arachidonsäure (AA)-induzierte Knochenresorption.
- Abbildung 5:: Calcitonin-ähnliche Wirkung des IFN-γ auf Parathormon (PTH)-induzierte Knochenresorption von kultivierten monotalen Mauskalvarien.
- Abbildung 6:: Einfluß von IFN-α,β auf die Knochenresorption von kultivierten neonatalen Mauskalvarien.
- Abbildung 7:: IFN-α,β und Parathormon (PTH)-Induktion der Knochenresorption von kultivierten neonatalen Mauskalvarien.
- Abbildung 8:: Thrombin-Bioassay; abgebildet sind der Zeitverlauf und die Dosiswirkungskurve der Thrombininduzierten Knochenresorption.
○ , Kontrolle; ● , 1U/ml; △ , 7 U/ml; □ , 14 U/ml; Insert: Dosis-Wirkungskurve von 0-42 U/ml Thrombin nach 72 Stunden Kulturzeit, gemessen am Calcium, das ins Kulturmedium freigesetzt wurde.

Die folgenden Beispiele sollen die Erfindung näher erläutern. Die an dem Modell erhaltenen Ergebnisse lassen sich auf jeden Warmblüter-Organismus übertragen.

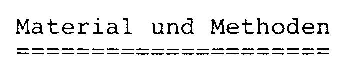

Die spezifische Aktivität des rekombinanten Maus-IFN-γ (aus E. coli) (Ernst-Boehringer-Institut für Arzneimittelforschung, Wien) betrug 1,3 x 10⁷ antivirale Einheiten per mg. IFN-γ war in RPMI-Medium gelöst und wurde bis zum Gebrauch in tiefgefrorenem Zustand aufbewahrt. IFN-α,β (aus Ehrlich Aszites-Zellen, induziert mit Newcastle Disease Virus, superinduziert mit Theophyllin war ein Produkt der Fa. ENZO Biochem., Inc. (N.Y.), (Ernst-Boehringer-Institut für Arzneimittelforschung).Die spezifische Aktivität der Charge Nr. 3-07001 betrug 44 x 10⁶U/mg Protein. Die Aktivität der von uns verwendeten Lösung wurde mit 60.000 Einheiten per ml bestimmt. Das zu Vergleichszwecken eingesetzte synthetische Salmcalcitonin (spezifische Aktivität 100 MRC-Einheiten/ml) war ein Produkt der Firma Sanabo (Wien). Parathormon wurde als synthetisches N-terminales Fragment 1-34 der Firma Bachem, Torrance, Kalifornien, verwendet. Prostaglandin E2 war das unter dem Namen Prostin E2 bekannte Produkt der Firma Upjohn. Bovines Thrombin ist eine Spezialität der Firma Hoffmann-LaRoche (Topostasin), Indomethacin wurde von Merck, Sharp und Dohme verwendet.

Neonatale Mauskalvarien können über längere Perioden (bis zu 96 Stunden und mehr) in Organkultur gehalten werden. Experimentelle Details sind von verschiedenen Autoren beschrieben worden (8,12,13). Im einzelnen wurden 4 bis 6 Tage alte SPF-Mäuse verwendet (Institut für Versuchstierzucht der Universität Wien, Himberg). Der Stamm trägt die Bezeichnung HIM:OF.

Die Präparation der Kalvarien geschieht unter sterilen Bedingungen (Laminar flow). Nach vorsichtiger Entfernung des anhaftenden Bindegewebes werden die Knochen in 1,0 ml Kulturmedium (siehe unten) in Eprouvetten transferiert. Nach Begasung mit 50 % O₂, 45 % N₂ und 5 % CO₂ werden diese fest verschlossen und dann während der ganzen Zeit der Organkultur in einer Rotationstrommel (Umlaufgeschwindigkeit 20 U mdr./h) bei 37^{o}C inkubiert.

Das Kulturmedium war Dulbecco's Modified Eagle's Medium (DMEM) von der Firma MA Bioproducts, Walkersville, Md. Dem Medium wurde L-Glutamin in einer Konzentration von 1,4 % und hitzeinaktiviertes Pferdeserum (15 %ig) zugesetzt. Das Pferdeserum (Gibco) wurde 45 min bei 56^{o} inaktiviert. Das komplette Medium wurde durch Sterilfilter (0,22 µm, Millipore) filtriert.

Nach 24 Stunden wurde das Kulturmedium mit den jeweiligen Zusätzen gewechselt und die Kultur dann bis zu insgesamt 72 oder max. auch 96 Stunden fortgesetzt.

Das Ausmaß der Knochenresorption kann durch Bestimmung des ins Medium abgegebene Calcium quantifiziert werden. Zu diesem Zwecke wurde zum Zeitpunkt 0, 24, 48, 72 oder 96 Stunden die Calciumkonzentration im Kulturmedium mit Hilfe einer Fluoreszenztitrationsmethode in einem Corning 940 Calciumanalyzer bestimmt.

Die Ergebnisse sind Mittelwerte aus 6 Kalvarien ± Mittleren Fehler des Mittelwertes. Statistisch signifikante Unterschiede zwischen den einzelnen Gruppen wurden durch den t-Test von Student festgestellt. Die Signifikanz wurde für einen Wert P<0,05 angenommen.

### Beispiel 1

### Wirkung von IFN-γ auf die basale Knochenresorption

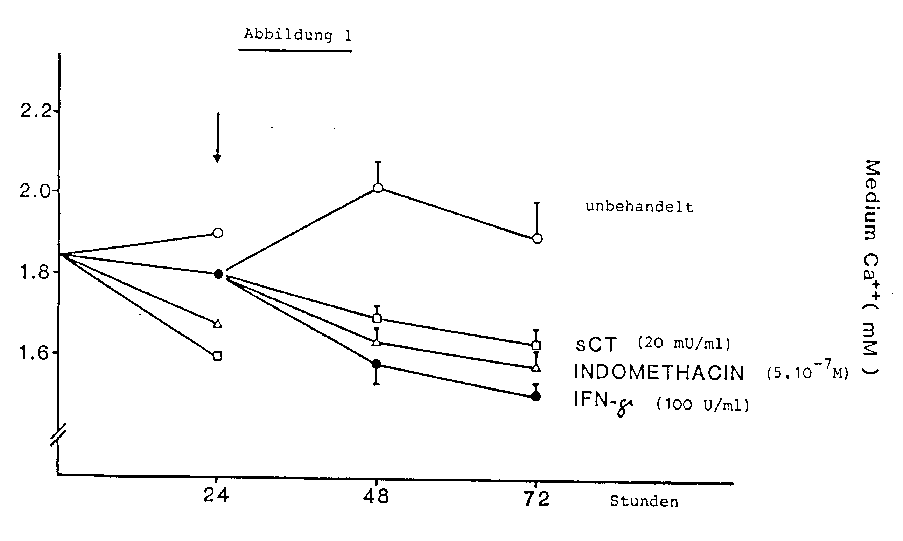

Werden die Knochen ohne jegliche Zusätze zum Medium kultiviert, dann zeigt ein leichter Anstieg der Calciumkonzentration im Medium an, daß der Knochen kontinuierlich resorbiert wird (Abb. 1, Kontrollen). Ursache dafür ist die endogene Bildung von Prostaglandinen (14,15). Tatsächlich kann durch Zusatz von Indomethacin, einem potenten Inhibitor der Prostaglandinsynthese (16), wie aus Abb. 1 zu ersehen ist, diese basale Resorption unterdrückt werden.

Zusatz von IFN-γ zum Kulturmedium in einer Konzentration von 100 U/ml bewirkt ebenfalls eine Hemmung der basalen Resorption, die in ihrem Ausmaß der Hemmwirkung von Calcitonin und auch dem suppressorischen Effekt von Indomethacin entspricht (siehe Abb.1). Die Ergebnisse sind in der folgenden Tabelle zusammengestellt.

| Medium Ca⁺⁺ in mMol/l (Mittelwerte aus 5 Knochen ± Standardfehler) | | | |
|---|---|---|---|
| Zusatz | 24 h | 48 h | 72 h |
| unbehandelt | 1.90±0.06 | 2.02±0.07 | 1.86±0.09 |
| sCt (Lachs-Calcitonin) (20 mU/ml) | 1.64±0.02 | 1.70±0.02 | 1.65±0.03 |
| Indomethacin (5 x 10⁻⁷ M) | 1.63±0.01 | 1.61±0.02 | 1.55±0.06 |
| IFN-γ (100 U/ml) | 1.80±0.03 | 1.69±0.02 | 1.52±0.04 |

### Beispiel 2

### Effekt von IFN-γ auf die durch Thrombin induzierte Resorption

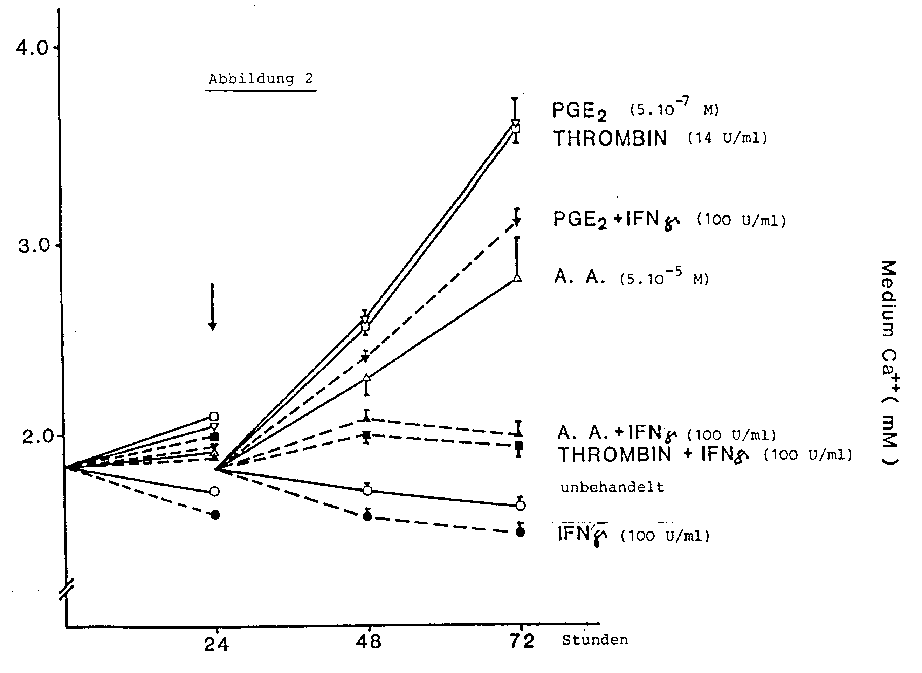

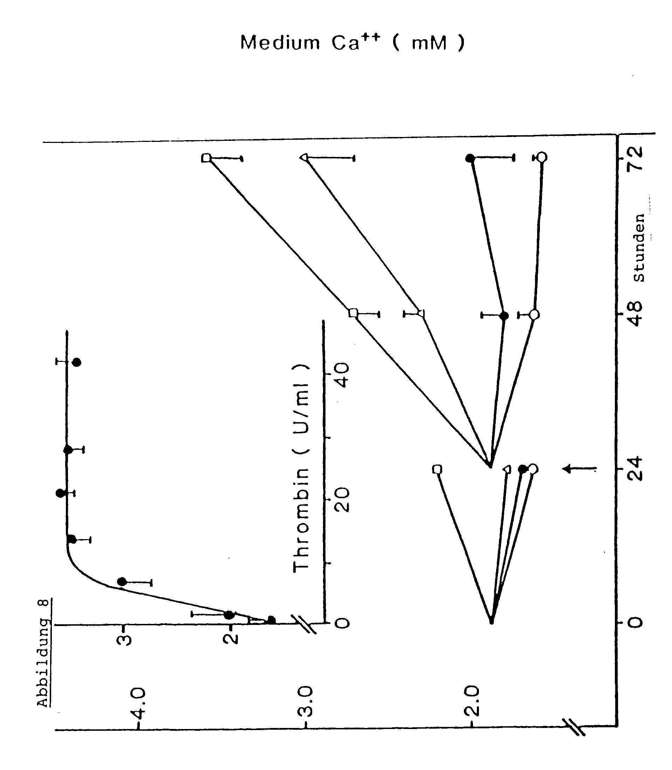

Zuerst wurde versucht zu klären, ob IFN-γ eventuell eine Hemmung der endogenen Prostaglandinproduktion in kultivierten Knochen bewirken könnte. Dazu wurde folgende Versuchsanordnung entwickelt: Ausgehend von der Beobachtung, daß der Zusatz von Thrombin zum Kulturmedium die durch Indomethacin hemmbare Knochenresorption verstärkt (18) und somit offenbar die endogene PG-Synthese stimuliert, wurde die Wirkung verschiedener Thrombinkonzentrationen auf die Resorption von Mäusekalvarien während einer Kulturzeit von 72 Stunden untersucht. Die Tabelle und Abb. 8 zeigen, daß Thrombinkonzentrationen im Bereich von 14-42 Einheiten/ml Kulturmedium eine maximale Freisetzung von Calcium in das Medium bewirken; das Ausmaß der Stimulierung der Resorptionsvorgänge durch Thrombin zeigt innerhalb eines Versuchs nur geringe relative Schwankungen und außerdem sind die gemessenen Absolutwerte der Calciumfreisetzung beim Vergleich der einzelnen Versuche gut reproduzierbar. Es sollte sich diese Versuchsanordnung daher gut zur Feststellung der Wirkung der Inhibitoren der PG-Synthese im Knochen eignen. Der genaue Wirkungsmechanismus von Thrombin ist nicht bekannt, doch wird angenommen, daß es durch seine proteolytische Aktivität (eventuell über Aktivierung anderer Proteasen) eine Phospholiphase aktivieren kann, die ihrerseits wieder aus Membranphospholipiden Arachidonsäure abspaltet. Dadurch steht fortwährend das Substrat für das Cyclooxygenasesystem zur Verfügung, so daß die endogene Synthese verschiedener Prostaglandine einer konstanten Stimulierung unterliegt. Die folgende Tabelle und Abb.2 zeigen, daß IFN-γ die durch Thrombin induzierte Knochenresorption vollständig unterdrückt. Der Effekt des Immuninterferons ist dabei durchaus mit der Wirkung von Indomethacin (5 x 10⁻⁷ M) zu vergleichen (17).

Thrombin-Bioassay:

| Medium Ca⁺⁺ in mMol/l (Mittelwerte aus 5 Knochen ± Standardfehler) | | | |
|---|---|---|---|
| Zusatz | 24 h | 48 h | 72 h |
| unbehandelt | 1.66±0.03 | 1.63±0.07 | 1.61±0.07 |
| Thrombin 1 U/ml | 1.67±0.03 | 1.81±0.09 | 2.01±0.31 |
| Thrombin 7 U/ml | 1.81±0.06 | 2.30±0.11 | 3.02±0.33 |
| Thrombin 14 U/ml | 2.13±0.08 | 2.66±0.11 | 3.56±0.21 |
| Thrombin 21 U/ml | 2.21±0.03 | 2.69±0.05 | 3.63±0.11 |
| Thrombin 28 U/ml | 2.28±0.02 | 2.71±0.06 | 3.61±0.12 |
| Thrombin 42 U/ml | 2.30±0.05 | 2.72±0.10 | 3.52±0.12 |

Die 72-Stunden-Werte sind im Insert für die Dosis-Wirkungskurve verwendet worden.

| Medium Ca⁺⁺ in mMol/l (Mittelwerte aus 5 Knochen ± Standardfehler) | | | |
|---|---|---|---|
| Zusatz | 24 h | 48 h | 72 h |
| unbehandelt | 1.72±0.03 | 1.71±0.02 | 1.63±0.04 |
| IFN-γ (100 U/ml) | 1.60±0.01 | 1.59±0.02 | 1.52±0.04 |
| PGE₂ (5x10⁻⁷ M) | 2.04±0.03 | 2.60±0.05 | 3.60±0.13 |
| PGE₂ (5x10⁻⁷ M) + IFN-γ (100 U/ml) | 1.95±0.04 | 2.40±0.01 | 3.10±0.06 |
| Thrombin (14 U/ml) | 2.11±0.07 | 2.62±0.05 | 3.62±0.06 |
| Thrombin (14 U/ml) + IFN-γ (100 U/ml) | 1.99±0.03 | 2.01±0.04 | 1.92±0.06 |
| Arachidonsäure (5x10⁻⁵M) | 1.92±0.05 | 2.35±0.10 | 2.85±0.27 |
| Arachidonsäure (5x10⁻⁵M) + IFN-γ (100 U/ml) | 1.92±0.09 | 2.01±0.03 | 1.93±0.03 |

### Beispiel 3

### Wirkung von IFN-γ auf den PG-Synthetasekomplex

Als nächstes wurde versucht festzustellen, ob IFN-γ auf die durch Thrombin ausgelöste Aktivierung weiterer Proteasen oder Phospholipasen einen Einfluß hat oder ob das Immuninterferon direkt mit dem Prostaglandinsynthetasekomplex interagiert. Zu diesem Zwecke wurde die Prostaglandinsynthese im kultivierten Knochen durch Zusatz von Arachidonsäure zum Kulturmedium stimuliert (15); diese Fettsäure wird - wie oben erwähnt - durch die Cyclooxygenasereaktion in verschiedene Prostaglandine umgewandelt. Aus ihrer osteolytischen Wirkung (Abb. 2) ist zu ersehen, daß auch exogene Arachidonsäure zur Prostaglandinsynthese in kultivierten Knochen verwendet wird. IFN-γ kann die durch Arachidonsäure stimulierte Knochenresorption in der Organkultur vollständig hemmen (Abb.2). Aus diesen Versuchen ergibt sich, daß IFN-γ direkt mit dem Prostaglandinsynthetasekomplex im Knochen interferieren kann.

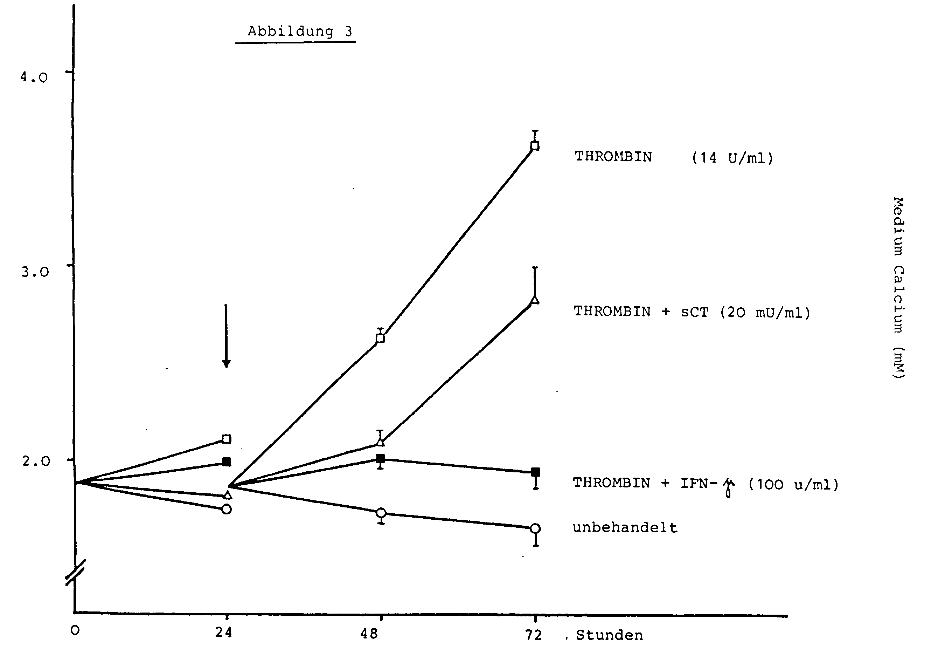

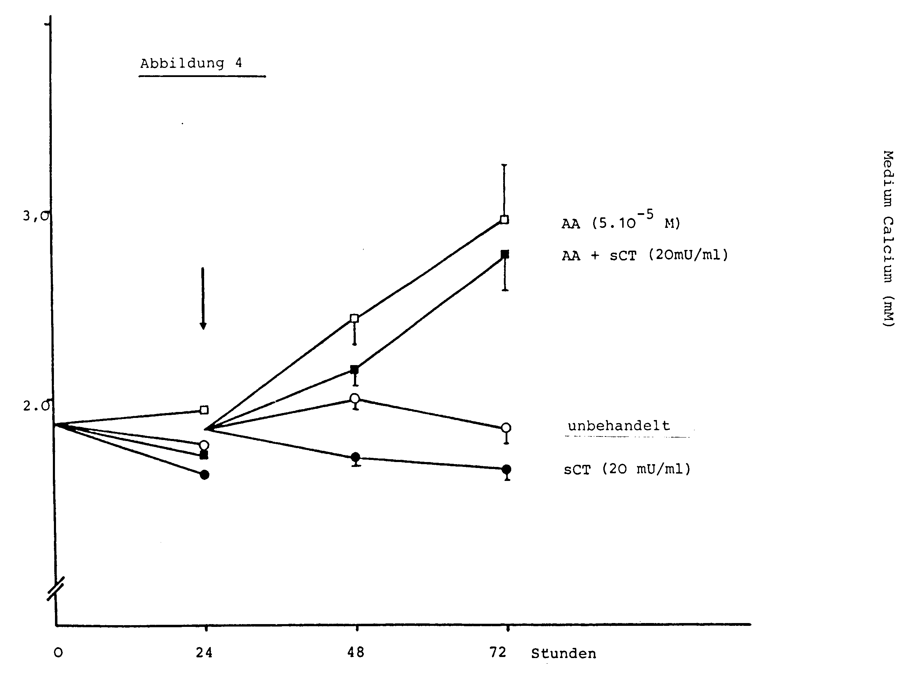

In diesem Zusammenhang soll hervorgehoben werden, daß Calitonin weder auf die durch Thrombin noch auf die durch Arachidonsäure induzierte Knochenresorption einen signifikanten Einfluß hat (siehe Abb. 3 und 4 und die folgenden Tabellen).

| Medium Ca⁺⁺ in mMmol/l (Mittelwerte aus 5 Knochen ± Standardfehler) | | | |
|---|---|---|---|
| Zusatz | 24 h | 48 h | 72 h |
| unbehandelt | 1.74±0.04 | 1.72±0.05 | 1.64±0.08 |
| Thrombin (14 U/ml) | 2.11±0.07 | 2.62±0.05 | 3.62±0.06 |
| Thrombin + sCt (20 mU/ml) | 1.82±0.01 | 2.08±0.08 | 2.82±0.19 |
| Thrombin (14 U/ml) + IFN-γ (100 U/ml) | 1.93±0.03 | 2.01±0.04 | 1.92±0.06 |

| Medium Ca⁺⁺ in mMol/l (Mittelwerte aus 5 Knochen ± Standardfehler) | | | |
|---|---|---|---|
| Zusatz | 24 h | 48 h | 72 h |
| unbehandelt | 1.78±0.03 | 2.01±0.04 | 1.85±0.06 |
| AA (5x10⁻⁵M) | 1.95±0.03 | 2.41±0.11 | 2.96±0.28 |
| AA (5x10⁻⁵M)+ sCt (20mU/ml) | 1.72±0.02 | 2.13±0.05 | 2.78±0.20 |
| sCt (20 mU/ml) | 1.64±0.02 | 2.18±0.06 | 1.65±0.03 |

### Beispiel 4

### Einfluß von IFN-γ auf die durch PGE₂ und PTH stimulierte Resorption

Nachdem nun festgestellt worden war, daß IFN-γ die Prostaglandinsynthese unterdrückt, war zu erwarten, daß sein Einfluß auf die osteolytische Wirkung von PGE₂, das dem Kulturmedium zugesetzt worden war, eher gering sein würde. Tatsächlich kann man aus Abb. 2 sehen, daß IFN-γ die osteolytische Wirkung von PGE₂ nur in geringem Ausmaß beeinflußt. Auffallend war jedoch die Tatsache, daß dieser Hemmeffekt doch größer war als es der Unterdrückung der basalen, durch endogene Prostaglandinsynthese vermittelten Knochenresorption entsprochen hätte. Untersucht wurde, ob IFN-γ zusätzlich zur Hemmung der Prostaglandinsynthese nicht auch Folgewirkungen der Prostaglandine selbst beeinflussen kann. Dazu gehört insbesondere die Aktivierung von Osteoklasten (8).

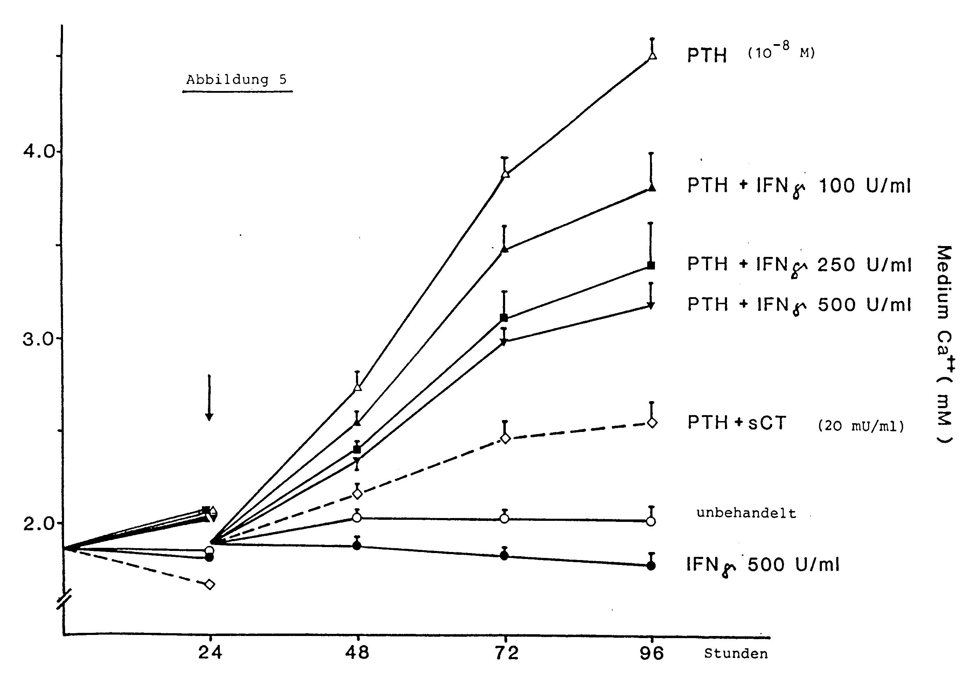

Auch Parathormon führt in der Organkultur zu einer Erhöhung der Zahl und der Aktivität von Osteoklasten, allerdings auf einem von Prostaglandinen unabhängigen Weg (7). Aus den in der Tabelle und in Abb. 5 dargestellten Versuchsergebnissen läßt sich ableiten, daß IFN-γ zusätzlich zu seiner Wirkung auf die Prostaglandinsynthese, eine calcitoninartige Wirkung auf die durch PTH induzierte Knochenresorption hat. Der Effekt von 20 mU/ml Salmcalcitonin auf die PTH-Wirkung ist ebenfalls zum Vergleich dargestellt.

| Medium Ca⁺⁺ in mMol/l (Mittelwerte aus 5 Knochen ± Standardfehler) | | | | |
|---|---|---|---|---|
| Zusatz | 24 h | 48 h | 72 h | 96 h |
| unbehandelt | 1.85±0.02 | 2.04±0.04 | 2.06±0.06 | 2.03±0.06 |
| IFN-γ (500 U/ml) | 1.89±0.03 | 1.89±0.04 | 1.84±0.03 | 1.78±0.03 |
| PTH (10⁻⁸M) | 2.08±0.05 | 2.75±0.07 | 3.89±0.06 | 4.51±0.06 |
| PTH (10⁻⁸M)+ IFN-γ (100 U/ml) | 2.07±0.02 | 2.55±0.06 | 3.49±0.11 | 3.82±0.17 |
| PTH(10⁻⁸M) + IFN-γ (250 U/ml) | 2.05±0.04 | 2.39±0.07 | 3.12±0.16 | 3.40±0.25 |
| PTH (10⁻⁸M)+ IFN-γ (500 U/ml) | 2.08±0.05 | 2.36±0.01 | 2.99±0.05 | 3.19±0.09 |
| PTH (10⁻⁸M)+ sCt (20 mU/ml) | 1.65±0.01 | 2.15±0.05 | 2.45±0.10 | 2.52±0.13 |

### Beispiel 5

### Einfluß von IFN-α,β auf basale und stimulierte Resorption

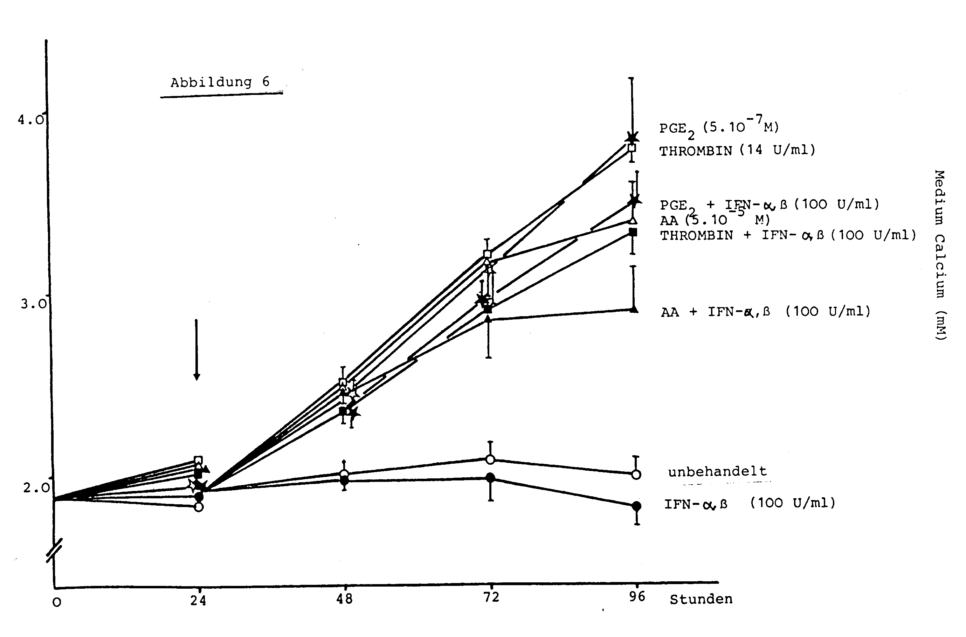

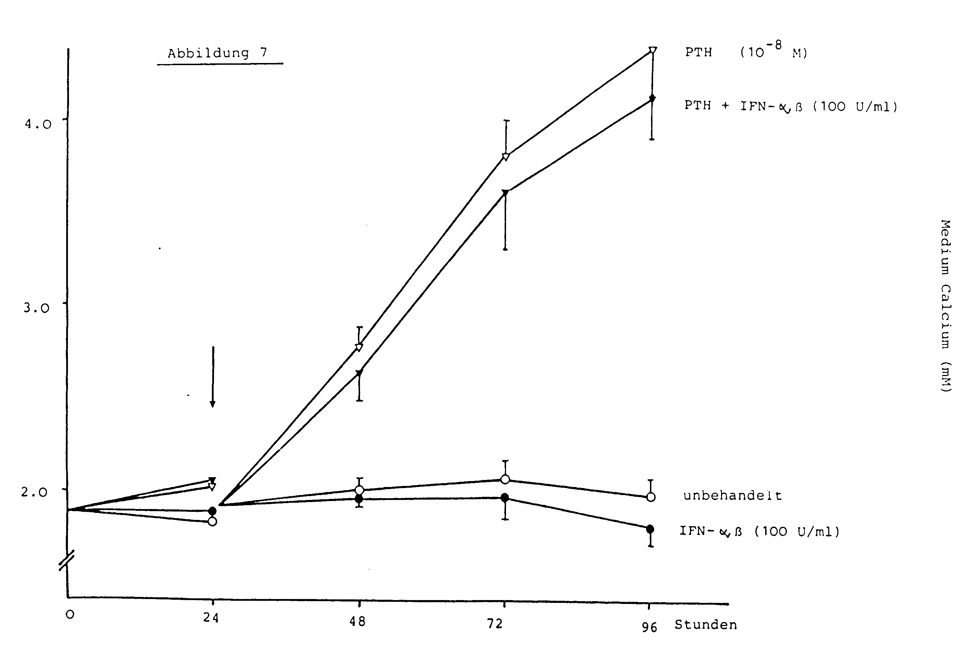

Um festzustellen, ob besonders die Wirkung auf die Prostaglandinsynthese spezifisch für das IFN-γ ist oder sie auch anderen Interferontypen zukommt, wurde die Versuchsserie mit einem α,β-Interferon (IFN-α,β) wiederholt. Abb. 6 zeigt, daß dieser Typ von Interferon - im Gegensatz zu IFN-γ - keine auffällige Hemmung der durch Thrombin oder Arachidonsäure induzierten Knochenresorption hervorrufen kann. In der verwendeten Konzentration (100 U/ml Medium) konnte auch keine Hemmung der von PTH bewirkten Knochenresorption durch IFN-α,β festgestellt werden (Abb. 7). Die Ergebnisse sind in den beiden folgenden Tabellen aufgeführt.

| Medium Ca⁺⁺ in mMol/l (Mittelwerte aus 5 Knochen ± Standardfehler) | | | | |
|---|---|---|---|---|
| Zusatz | 24 h | 48 h | 72 h | 96 h |
| unbehandelt | 1.85±0.03 | 2.02±0.04 | 2.09±0.08 | 2.01±0.08 |
| IFNα/β (100 U/ml) | 1.88±0.08 | 2.02±0.10 | 1.98±0.12 | 1.92±0.12 |
| PGE₂ (5x10⁻⁷ M) | 1.98±0.05 | 2.44±0.08 | 3.13±0.19 | 3.91±0.25 |
| PGE₂ (5x10⁻⁷M) + IFNα/β (100 U/ml) | 1.96±0.05 | 2.38±0.06 | 2.96±0.11 | 3.49±0.16 |
| Thrombin (14 U/ml) | 2.08±0.04 | 2.50±0.01 | 3.20±0.06 | 3.81±0.08 |
| Thrombin (14 U/ml) + IFNα/β (100 U/ml) | 2.04±0.02 | 2.36±0.02 | 2.89±0.07 | 3.31±0.13 |
| AA. (5x10⁻⁷ M) | 2.07±0.03 | 2.50±0.08 | 3.15±0.16 | 3.39±0.27 |
| AA. (5x10⁻⁷ M) + IFNα/β (100 U/ml) | 2.03±0.04 | 2.46±0.07 | 2.84±0.11 | 2.89±0.22 |

| Medium Ca⁺⁺ in mMol/l (Mittelwerte aus 5 Knochen ± Standardfehler) | | | | |
|---|---|---|---|---|
| Zusatz | 24 h | 48 h | 72 h | 96 h |
| unbehandelt | 1.85±0.03 | 2.02±0.04 | 2.09±0.08 | 2.01±0.08 |
| IFNα/β (100 U/ml) | 1.88±0.08 | 2.02±0.10 | 1.98±0.12 | 1.92±0.12 |
| PTH (10⁻⁸M) | 2.04±0.04 | 2.78±0.09 | 3.91±0.22 | 4.42±0.39 |
| PTH (10⁻⁸M) + IFNα/β (100 U/ml) | 1.97±0.06 | 2.64±0.11 | 3.62±0.23 | 4.14±0.27 |

### Literatur

1. R. Baron, A. Vignery and M. Horowitz: Lympnocytes, Macrophages and the Regulation of Bone Remodeling. In: Bone and Mineral Research, Annual 2 (W.A. Peck, ed.), p. 175, Elsevier 1983
2. L.G. Raisz and B.E. Kream: Hormonal Control of Skeletal Growth. Ann. Rev. Physiol. 43:225, 1981
3. G.L. Wong: Paracrine interactions in bone-secreted products of osteoblasts permit osteoclasts to respond to parathyroid hormone. J. Biol. Chem. 259:4019-4022, 1984
4. G.A. Howard, B.L. Bottemiller, R.T. Turner, J.I. Rader and D.J. Baylink: Parathyroid hormone stimulates bone formation and resorption in organ culture: Evidence for a coupling mechanism. Proc. Natl. Acad. Sci. USA 78:3204-3208, 1981
5. L.V. Avioli: Osteoporosis. In: Bone and Mineral Research, Annual 1 (W.A. Peck, ed.), p. 280-318, Elsevier, 1983
6. G.A. Rodan and T.J. Martin: Role of Osteoblasts in Hormonal Control of Bone Resorption - A Hypothesis. Calcif. Tissue Int. 33:349-351, 1981
7. R.S. Feldman, N.S. Krieger and A.H. Tashjian: Effects of Parathyroid Hormone and Calcitonin on Osteoclast Formation in Vitro. Endocrinology 107:1137-1143, 1980
8. S.H.Shelling, H.J. Wolfe and A.H. Tashjian: Role of the Osteoclast in Prostaglandin E₂-Stimulated Bone Resorption. Lab. Invest 42:290-295, 1980
9. J.N. Beresford et al.: The effects of monocyte-conditioned medium and interleukin 1 on the synthesis of collagenous and non-collagenous proteins by mouse bone and human bone cells in vitro. Biochim. Biophys. Acta 801:58-65, 1984
10. R.S. Bockman and M.A. Repo: Lymphokine-mediated bone resorption requires endogenous prostaglandin synthesis. J. Exp. Med. 154:529-534, 1981
11. J.B. Weinberg, M.M. Hobbs and M.A. Miskounis: Recombinant human γ-interferon induces human monocyte polykaryon formation. Proc. Natl. Acad. Sci. USA 81:4554-4557, 1984
12. N.S. Krieger and P.H. Stern: Interaction between amrinone and parathyroid hormone on bone in culture. Am. J. Physiol. 243:E499-504, 1982
13. P.H. Stern and N.S. Krieger: Comparison of Fetal Rat Limb Bones and Neonatal Mouse Calvaria: Effects of Parathyroid Hormone and 1,25-Dihydroxyvitamin D₃. Calcif. Tissue Int. 35:172-176, 1983
14. J.M. Katz, S.J.M. Skinner, T. Wilson and D.H. Gray: The In Vitro Effect of Indomethacin on Basal Bone Resorption, on Prostaglandin Production and on the Response to Added Prostaglandins. Prostaglandins 26:545-
15. J.M. Katz, T. Wilson, S.J.M. Skinner and D.H. Gray: Bone Resorption and Prostaglandin Production by Mouse Calvaria in Vitro: Response to Exogenous Prostaglandins and Their Precursor Fatty Acids. Prostaglandins 22:537-543, 1981
16. J.R. Vane: Inhibition of Prostaglandin-Synthesis as a Mechanism of Action for Aspirin-like Drugs. Nature N.B. 231:232-23 , 1971
17. O. Hoffmann, K. Klaushofer, K. Koller and M. Peterlik: Prostaglandin-related bone resorption in cultured neonatal mouse calvaria: Evaluation of biopotency of nonsteroidal anti-inflammatory drugs. Manuscript in preparation
18. G.T. Gustafson and U. Lerner: Thrombin, a stimulator of bone resorption. Bioscience Reports 3:255-261, 1983
19. R.L. Jilka and J.W. Hamilton: Inhibition of parathromone stimulated bone resorption by type I interferon. Biochem. Biophys. Res. Comm. 120:553-558, 1984
20. D.A. Stringfellow and R. Brideau: Interferons and Prostaglandins. In: Interferon, vol. 2 (J. Vil 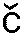 ek and E. De Maeyer, eds.), p. 147-163, Elsevier 1984
21. C.A. Dinarello et al.: Mechanisms of Fever Induced by Recombinant Human Interferon. J. Clin. Invest. 74:906-913, 1984
22. R.J. Flowers: Drugs which inhibit prostaglandin biosynthesis. Pharmacol. Rev. 26:33-67, 1974
23. O.S. Nilsson et al.: Effect of Interferon on Heterotopic New Bone Formation in Mice. J. Interferon Res. 4:135-140, 1984
24. R.M. Friedman and S.N. Vogel: Interferons with Special Emphasis on the Immune System. In: Advances in Immunology, vol. 34 (F.J. Dixon and H.G. Kunkel, eds.), p. 97-140, Academic Press, New York, 1983
25. M.R. Warner et al.: Ametantrone inhibits prostaglandinmediated resorption in bone organ culture. Prostaglandins 28:469-476, 1984
26. O.S. Atik, A. Surat and M.T. Goegues: Prostaglandin E₂-like activity and senile osteoporosis. Prostaglandins Leukotrienes Med. 11:105-107, 1983
27. T. Fujik et al.: T Lymphocyte subsets in Osteoporosis. Effect of 1-Alpha-Hydroxyvitamin D₃. Mineral Electrolyte Metab. 10:375-378, 1984
28. Round Table Discussion: Pathogenesis and Treatment of Postmenopausal Osteoporosis. Calcif. Tissue Int. 35:708-711, 1983

## Patentansprüche

1. Verwendung von IFN-γ zur Herstellung eines Arzneimittels zur Regulierung der beim Knochenauf- und -abbau ablaufenden Prozesse.

2. Verwendung von IFN-γ nach Anspruch 1 zur Behandlung von systemischen oder lokalen Knochenerkrankungen mit entzündlicher oder nicht-entzündlicher Genese (degenerative Erkrankungen).

3. Verwendung von IFN-γ nach Anspruch 1 zur Behandlung von krankhaftem Knochenschwund.

4. Verwendung von IFN-γ nach Anspruch 1 zur Behandlung von Knochenerkrankungen, die durch lokalen oder generalisierten Abbau des Knochengewebes hervorgerufen werden.

5. Verwendung von IFN-γ nach Anspruch 1 zur Hemmung von Vorgängen, die zur erhöhten Resorption von Knochen führen.

6. Verwendung von IFN-γ nach Anspruch 1 zur Hemmung der endogenen Synthese von Prostaglandinen in Knochen.

7. Verwendung von IFN-γ nach Anspruch 1 und 2 zur Hemmung der endogenen Synthese und osteolytischen Wirkung von Prostaglandinen in Kombination mit nicht-steroidalen Enzündungshemmern mit geringer oder fehlender Hemmwirkung auf die Prostaglandin-Synthese im Knochen.

8. Verwendung von IFN-γ nach Anspruch 1 zur Hemmung der Osteoklastenaktivität.

9. Verwendung von IFN-γ nach Anspruch 1 zur Behandlung von allen durch Parathormon hervorgerufenen Osteodystrophien insbesondere bei renal bedingten Osteopathien.

10. Verwendung von IFN-γ nach Anspruch 1 und 9 zur adjuvanten Therapie bei progredienter Niereninsuffizienz zur Unterdrückung der durch Parathormon hervorgerufenen Knochenresorption bei gleichzeitiger immunsuppressiver Wirkung im Falle von autoimmun bedingten Glomerulonephriditen.

11. Verwendung von IFN-γ nach Anspruch 1 zur Behandlung aller Formen und Stadien der Osteoporose.

12. Verwendung von IFN-γ nach Anspruch 1 zur Behandlung des Morbus Paget allein oder in Kombination mit Diphosphonaten.

13. Verwendung von IFN-γ nach Anspruch 1 zur Behandlung von Erkrankungen des rheumatischen Formenkreises, insbesondere der rheumatischen Arthritis (primär chronische Polyarthritis).

14. Verwendung von IFN-γ nach Anspruch 1 zur Behandlung von Kiefer- und Zahnerkrankungen mit gesteigertem Knochenschwund.

15. Verwendung von IFN-γ nach Anspruch 1 zur Behandlung der Hypercalcämie bei Prostaglandin-synthetisierenden Tumoren (Malignomen).

16. Verwendung von IFN-γ nach einem der vorhergehenden Ansprüche in Kombination mit bekannten Wirkstoffen zur Behandlung von Knochenerkrankungen.

## Claims

1. Use of IFN-γ for the preparation of a pharmaceutical composition for regulating the processes occurring in bone formation and decomposition.

2. Use of IFN-γ according to claim 1 for the treatment of systemic or local bone diseases of inflammatory or non-inflammatory origin (degenerative diseases).

3. Use of IFN-γ according to claim 1 for the treatment of pathological bone loss.

4. Use of IFN-γ according to claim 1 for the treatment of bone diseases caused by local or generalised decomposition of the bone tissue.

5. Use of IFN-γ according to claim 1 for inhibiting processes which lead to increased resorption of bones.

6. Use of IFN-γ according to claim 1 for inhibiting the endogenous synthesis of prostaglandins in bones.

7. Use of IFN-γ according to claims 1 and 2 for inhibiting the endogenous synthesis and osteolytic activity of prostaglandins in conjunction with non-steroidal anti-inflammatory agents with little or no inhibiting effect on prostaglandin synthesis in bone.

8. Use of IFN-γ according to claim 1 for inhibiting osteoclast activity.

9. Use of IFN-γ according to claim 1 for the treatment of all osteodystrophies caused by parathormone, particularly in renally caused osteopathies.

10. Use of IFN-γ according to claims 1 and 9 for additional therapy in progressive kidney failure for suppressing the parathormone-induced bone resorption with a simultaneous immuno-suppressant activity in the case of glomerulonephritides caused by autoimmune response.

11. Use of IFN-γ according to claim 1 for the treatment of all forms and stages of osteoporosis.

12. Use of IFN-γ according to claim 1 for the treatment of Paget's disease, on its own or in conjunction with diphosphonates.

13. Use of IFN-γ according to claim 1 for the treatment of diseases of the rheumatic type, particularly rheumatoid arthritis (primary chronic polyarthritis).

14. Use of IFN-γ according to claim 1 for the treatment of jaw and tooth diseases with increased bone loss.

15. Use of IFN-γ according to claim 1 for the treatment of hypercalcaemia in prostaglandin-synthesising tumours (malignomas).

16. Use of IFN-γ according to one of the preceding claims in conjunction with known active substances for the treatment of bone disease.

## Revendications

1. Utilisation de IFN-γ pour la préparation d'un médicament pour la régulation des processus qui surviennent lors de la formation et de la dégradation des os.

2. Utilisation de IFN-γ selon la revendication 1 pour le traitement des maladies osseuses systémiques ou locales à genèse inflammatoire ou non inflammatoire (maladies dégénératives).

3. Utilisation de IFN-γ selon la revendication 1 pour le traitement de l'atrophie osseuse pathologique.

4. Utilisation de IFN-γ selon la revendication 1 pour le traitement des maladies osseuses qui sont provoquées par une dégradation locale ou généralisée du tissu osseux.

5. Utilisation de IFN-γ selon la revendication 1 pour l'inhibition des processus qui entraînent une plus forte résorption des os.

6. Utilisation de IFN-γ selon la revendication 1 pour l'inhibition de la synthèse endogène des prostaglandines dans les os.

7. Utilisation de IFN-γ selon les revendications 1 et 2 pour l'inhibition de la synthèse endogène et de l'effet ostéolytique des prostaglandines en combinaison avec des anti-inflammatoires non stéroïdiens ayant un effet inhibiteur faible ou nul sur la synthèse des prostaglandines dans les os.

8. Utilisation de IFN-γ selon la revendication 1 pour l'inhibition de l'activité des ostéoclastes.

9. Utilisation de IFN-γ selon la revendication 1 pour le traitement de toutes les ostéodystrophies provoquées par l'hormone parathyroïdienne, en particulier dans le cas des ostéopathies d'origine rénale.

10. Utilisation de IFN-γ selon les revendications 1 et 9 pour la thérapie adjuvante dans le cas d'une insuffisance rénale évolutive pour réprimer la résorption des os provoquée par l'hormone parathyroïdienne avec un effet immunosuppresseur simultané dans le cas des glomérulonéphrites d'origine auto-immune.

11. Utilisation de IFN-γ selon la revendication 1 pour le traitement de toutes les formes et de tous les stades de l'ostéoporose.

12. Utilisation de IFN-γ selon la revendication 1 pour le traitement de la maladie osseuse de Paget seul ou en combinaison avec des diphosphonates.

13. Utilisation de IFN-γ selon la revendication 1 pour le traitement des maladies de type rhumatoïde, en particulier de la polyarthrite rhumatoïde (polyarthrite chronique primaire).

14. Utilisation de IFN-γ selon la revendication 1 pour le traitement des maladies des mâchoires et des dents à atrophie accentuée.

15. Utilisation de IFN-γ selon la revendication 1 pour le traitement de l'hypercalcémie dans le cas des tumeurs synthétisant des prostaglandines (malignomes).

16. Utilisation de IFN-γ selon l'une des revendications précédentes en combinaison avec des principes actifs connus pour le traitement des maladies osseuses.
